(19) Europäisches Patentamt / European Patent Office / Office européen des brevets

(11) **EP 3 588 332 A1**

(12) # EUROPEAN PATENT APPLICATION

(43) Date of publication:
   **01.01.2020 Bulletin 2020/01**

(51) Int Cl.:
   ***G06F 17/50*** *(2006.01)*

(21) Application number: **18196558.3**

(22) Date of filing: **25.09.2018**

(84) Designated Contracting States:
   **AL AT BE BG CH CY CZ DE DK EE ES FI FR GB GR HR HU IE IS IT LI LT LU LV MC MK MT NL NO PL PT RO RS SE SI SK SM TR**
   Designated Extension States:
   **BA ME**
   Designated Validation States:
   **KH MA MD TN**

(30) Priority: **28.06.2018 CN 201810690706**

(71) Applicant: **Dong Han New Energy Automotive Technology Co., Ltd**
   **201801 Shanghai (CN)**

(72) Inventor: **Wang, Wei**
   **Shanghai, 201801 (CN)**

(74) Representative: **Soria Parra, Manuel**
   **Meissner Bolte**
   **Patentanwälte Rechtsanwälte**
   **Partnerschaft mbB**
   **Widenmayerstraße 47**
   **80538 München (DE)**

(54) ## METHOD AND DEVICE FOR PREDICTING FIBER ORIENTATION

(57)   The present invention relates to a method and a device for predicting fiber orientation. The method includes: obtaining a mechanical property parameter value of a fiber composite material; obtaining a change value of a fiber property of the fiber composite material after deformation of the fiber composite material, according to the mechanical property parameter value; and predicting fiber orientation in a forming process of the fiber composite material according to the change value of the fiber property and a preset fiber orientation model, the preset fiber orientation model indicating a mapping relationship between the change value of the fiber property and the fiber orientation after the deformation of the fiber composite material.

OBTAIN A MECHANICAL PROPERTY PARAMETER VALUE OF A FIBER COMPOSITE MATERIAL — S101

OBTAIN A CHANGE VALUE OF A FIBER PROPERTY OF THE FIBER COMPOSITE MATERIAL AFTER DEFORMATION OF THE FIBER COMPOSITE MATERIAL, ACCORDING TO THE MECHANICAL PROPERTY PARAMETER VALUE — S102

PREDICT FIBER ORIENTATION IN A FORMING PROCESS OF THE FIBER COMPOSITE MATERIAL ACCORDING TO THE CHANGE VALUE OF THE FIBER PROPERTY AND A PRESET FIBER ORIENTATION MODEL, THE PRESET FIBER ORIENTATION MODEL INDICATING A MAPPING RELATIONSHIP BETWEEN THE CHANGE VALUE OF THE FIBER PROPERTY AND THE FIBER ORIENTATION AFTER THE DEFORMATION OF THE FIBER COMPOSITE MATERIAL — S103

Fig. 1

**Description**

**TECHNICAL FIELD**

The present invention relates to the technical field of fiber orientation prediction, and more particularly to a method and a device for predicting fiber orientation.

**BACKGROUND**

Fiber composite material is an important engineering material, which is widely used in engineering and has broad development prospect. The performance of fiber composite material is closely related to a distribution state of interior fiber orientation. Therefore, prediction of fiber orientation in the formation of fiber composite material is of great significance.

At present, finite element software is usually used in predicting fiber orientation, for determining directions of the strongest and weakest fiber orientations according to main strain directions in individual elements of a finite element model; and fiber orientation is predicted according to the magnitudes of a first, second and third main strain of each element. However, this method is directed to short fibers. Since the fibers in short fiber composite material are completely randomly blended or approximately randomly blended on a microstructure scale, a three-dimensionally woven short fiber reinforced composite material is an isotropic material.

However, a three-dimensionally woven long fiber composite material is a typical anisotropic material, and therefore, the above method is not suitable for predicting fiber orientation of the three-dimensionally woven long fiber composite material.

**SUMMARY**

In order to overcome the problem in the related art, embodiments of the present invention provide a method and a device for predicting fiber orientation. The technical solutions are as follows:

According to a first aspect of one embodiment of the present invention, there is provided a method for predicting fiber orientation. The method includes:

obtaining a mechanical property parameter value of a fiber composite material;

obtaining a change value of a fiber property of the fiber composite material after deformation of the fiber composite material, according to the mechanical property parameter value; and

predicting fiber orientation in a forming process of the fiber composite material according to the change value of the fiber property and a preset fiber orientation model, the preset fiber orientation model indicating a mapping relationship between the change value of the fiber property and the fiber orientation after the deformation of the fiber composite material.

The technical solution provided in embodiment of the present invention may have the following beneficial effects: obtaining a mechanical property parameter value of a fiber composite material; obtaining a change value of a fiber property of the fiber composite material after deformation of the fiber composite material, according to the mechanical property parameter value; and predicting fiber orientation in a forming process of the fiber composite material according to the change value of the fiber property and a preset fiber orientation model, the preset fiber orientation model indicating a mapping relationship between the change value of the fiber property and the fiber orientation after the deformation of the fiber composite material. The present invention introduces a preset fiber orientation model for indicating a mapping relationship between the change value of the fiber property and the fiber orientation after deformation of the fiber composite material, so that fiber orientation in a forming process of the fiber composite material can be predicted from the fiber change value as obtained.

In an embodiment, said obtaining a change value of a fiber property of the fiber composite material after deformation of the fiber composite material, according to the mechanical property parameter value includes:

obtaining a length change value of a fiber in the fiber composite material along respective coordinate axes in a predetermined Cartesian coordinate system and a change value of an angle between a warp fiber and a weft fiber in the fiber composite material in the predetermined Cartesian coordinate system, after the deformation of the fiber composite material; and

obtaining a length change value of a fiber in the fiber composite material along respective coordinate axes in a relative coordinate system and a change value of an angle between a warp fiber and a weft fiber in the fiber composite material in the relative coordinate system, after the deformation of the fiber composite material.

In an embodiment, prior to said predicting fiber orientation in the fiber composite material according to the change value of the fiber property and a preset fiber orientation model, the method further includes:

obtaining a Cartesian component of stress-strain tensor according to a change value of the fiber in a preset Cartesian coordinate system, the change value of the fiber in the preset Cartesian coordinate system including: a length change value of the fiber along respective coordinate axes in the predetermined Cartesian coordinate system, and a change value of an angle between a warp fiber and a weft fiber in the composite material in the predetermined Cartesian coordinate system;

obtaining a relative coordinate component of the stress-strain tensor according to a change value of the fiber in a relative coordinate system, the change value of the fiber in the relative coordinate system including: a length change value of the fiber along respective coordinate axes in the relative coordinate system, and a change value of an angle between a warp fiber and a weft fiber in the composite material in the relative coordinate system; and

obtaining the preset fiber orientation model according to a relationship among a Cartesian component of the stress-strain tensor, a relative coordinate component of the stress-strain tensor, and fiber orientation.

In an embodiment, the Cartesian component of the stress-strain tensor is expressed as:

$$\left\{ \begin{array}{c} d\varepsilon_{11} \\ d\varepsilon_{22} \\ d\gamma_{12} \end{array} \right\}$$

where $d\varepsilon_{11}$ represents a length change value of the fiber along a first coordinate axis in the preset Cartesian coordinate system; $d\varepsilon_{22}$ represents a length change value of the fiber along a second coordinate axis in the preset Cartesian coordinate system; and $d\gamma_{12}$ represents a change value of an angle between a warp fiber and a weft fiber in the composite material in the preset Cartesian coordinate system.

In an embodiment, the relative coordinate component of the stress-strain tensor is expressed as:

$$\left\{ \begin{array}{c} d\bar{\varepsilon}_{11} \\ d\bar{\varepsilon}_{22} \\ d\bar{\gamma}_{12} \end{array} \right\}$$

where $d\bar{\varepsilon}_{11}$ represents a length change value of the fiber along a first coordinate axis in the relative coordinate system; $d\bar{\varepsilon}_{22}$ represents a length change value of the fiber along a second coordinate axis in the relative coordinate system; and $d\bar{\gamma}_{12}$ represents a change value of an angle between a warp fiber and a weft fiber in the composite material in the relative coordinate system.

In an embodiment, the relationship among the Cartesian component of the stress-strain tensor, the relative coordinate component of the stress-strain tensor and the fiber orientation includes:

the relative coordinate component of the stress-strain tensor is a product of the Cartesian component of the stress-strain tensor, and a preset trigonometric function value corresponding to an angular value of the fiber orientation.

In an embodiment, said the relative coordinate component of the stress-strain tensor being a product of the Cartesian component of the stress-strain tensor, and a preset trigonometric function value corresponding to an angular value of the fiber orientation includes:

$$\left\{ \begin{array}{c} d\bar{\varepsilon}_{11} \\ d\bar{\varepsilon}_{22} \\ d\bar{\gamma}_{12} \end{array} \right\} = \left[ \begin{array}{ccc} \cos^2 \alpha & \sin^2 \alpha & \sin \alpha \cos \alpha \\ \cos^2(\alpha + \theta) & \sin^2(\alpha + \theta) & \sin(\alpha + \theta)\cos(\alpha + \theta) \\ 2 \cos \alpha \cos(\alpha + \theta) & 2 \sin \alpha \sin(\alpha + \theta) & \sin(2\alpha + \theta) \end{array} \right] \left\{ \begin{array}{c} d\varepsilon_{11} \\ d\varepsilon_{22} \\ d\gamma_{12} \end{array} \right\}$$

where the $\alpha$ represents an angle change value of the angle between the fiber and the first coordinate axis in the preset Cartesian coordinate system; and the $\theta$ represents an angle value of the fiber orientation.

In an embodiment, said obtaining a mechanical property parameter value of a fiber composite material includes:
obtaining the mechanical property parameter value of the fiber composite material by using preset simulation software.
According to a second aspect of one embodiment of the present invention, there is provided a device for predicting fiber orientation. The device includes:

a first obtaining module, configured to obtain a mechanical property parameter value of a fiber composite material;

a second obtaining module, configured to obtain a change value of a fiber property of the fiber composite material after deformation of the fiber composite material, according to the mechanical property parameter value obtained by the first obtaining module; and

a predicting module, configured to predict fiber orientation in a forming process of the fiber composite material according to the change value of the fiber property obtained by the second obtaining module and a preset fiber orientation model, the preset fiber orientation model indicating a mapping relationship between the change value of the fiber property and the fiber orientation after the deformation of the fiber composite material.

In an embodiment, the second obtaining module includes a first obtaining sub-module and a second obtaining sub-module,
the first obtaining sub-module is configured to obtain a length change value of a fiber in the fiber composite material along respective coordinate axes in a predetermined Cartesian coordinate system and a change value of an angle between a warp fiber and a weft fiber in the fiber composite material in the predetermined Cartesian coordinate system, after the deformation of the fiber composite material; and
the second obtaining sub-module is configured to obtain a length change value of a fiber in the fiber composite material along respective coordinate axes in a relative coordinate system and a change value of an angle between a warp fiber and a weft fiber in the fiber composite material in the relative coordinate system, after the deformation of the fiber composite material.
In an embodiment, the device further includes a third obtaining module, a fourth obtaining module, and a fifth obtaining module,
the third obtaining module is configured to obtain a Cartesian component of stress-strain tensor according to a change value of the fiber in a preset Cartesian coordinate system, the change value of the fiber in the preset Cartesian coordinate system including: a length change value of the fiber along respective coordinate axes in the predetermined Cartesian coordinate system, and a change value of an angle between a warp fiber and a weft fiber in the composite material in the predetermined Cartesian coordinate system;
the fourth obtaining module is configured to obtain a relative coordinate component of the stress-strain tensor according to a change value of the fiber in a relative coordinate system, the change value of the fiber in the relative coordinate system including: a length change value of the fiber along respective coordinate axes in the relative coordinate system, and a change value of an angle between a warp fiber and a weft fiber in the composite material in the relative coordinate system; and
the fifth obtaining module is configured to obtain the preset fiber orientation model according to a relationship among a Cartesian component of the stress-strain tensor obtained by the third obtaining module, a relative coordinate component of the stress-strain tensor obtained by the fourth obtaining module, and fiber orientation.
In an embodiment, the Cartesian component of the stress-strain tensor is expressed as:

$$\left\{ \begin{array}{c} d\varepsilon_{11} \\ d\varepsilon_{22} \\ d\gamma_{12} \end{array} \right\}$$

where $d\varepsilon_{11}$ represents a length change value of the fiber along a first coordinate axis in the preset Cartesian coordinate system; $d\varepsilon_{22}$ represents a length change value of the fiber along a second coordinate axis in the preset Cartesian coordinate system; and $d\gamma_{12}$ represents a change value of an angle between a warp fiber and a weft fiber in the composite material in the preset Cartesian coordinate system.
In an embodiment, the relative coordinate component of the stress-strain tensor is expressed as:

$$\left\{ \begin{array}{c} \mathrm{d}\bar{\varepsilon}_{11} \\ \mathrm{d}\bar{\varepsilon}_{22} \\ \mathrm{d}\bar{\gamma}_{12} \end{array} \right\}$$

where $\mathrm{d}\bar{\varepsilon}_{11}$ represents a length change value of the fiber along a first coordinate axis in the relative coordinate system; $\mathrm{d}\bar{\varepsilon}_{22}$ represents a length change value of the fiber along a second coordinate axis in the relative coordinate system; and $\mathrm{d}\bar{\gamma}_{12}$ represents a change value of an angle between a warp fiber and a weft fiber in the relative coordinate system.

In an embodiment, the relationship among the Cartesian component of the stress-strain tensor, the relative coordinate component of the stress-strain tensor and the fiber orientation includes:

the relative coordinate component of the stress-strain tensor is a product of the Cartesian component of the stress-strain tensor and a preset trigonometric function value corresponding to an angular value of the fiber orientation.

In an embodiment, said the relative coordinate component of the stress-strain tensor being a product of the Cartesian component of the stress-strain tensor, and a preset trigonometric function value corresponding to an angular value of the fiber orientation includes:

$$\left\{ \begin{array}{c} \mathrm{d}\bar{\varepsilon}_{11} \\ \mathrm{d}\bar{\varepsilon}_{22} \\ \mathrm{d}\bar{\gamma}_{12} \end{array} \right\} = \left[ \begin{array}{ccc} \cos^2 \alpha & \sin^2 \alpha & \sin \alpha \cos \alpha \\ \cos^2(\alpha + \theta) & \sin^2(\alpha + \theta) & \sin(\alpha + \theta)\cos(\alpha + \theta) \\ 2 \cos \alpha \cos(\alpha + \theta) & 2 \sin \alpha \sin(\alpha + \theta) & \sin(2\alpha + \theta) \end{array} \right] \left\{ \begin{array}{c} \mathrm{d}\varepsilon_{11} \\ \mathrm{d}\varepsilon_{22} \\ \mathrm{d}\gamma_{12} \end{array} \right\}$$

where the $\alpha$ represents an angle change value of the angle between the fiber and the first coordinate axis in the preset Cartesian coordinate system; the $\theta$ represents an angle value of the fiber orientation.

In an embodiment, the first obtaining module includes a third obtaining sub-module;

the third obtaining sub-module is configured to obtain the mechanical property parameter value of the fiber composite material by using preset simulation software.

It is to be understood that both the foregoing general description and the following detailed description are exemplary and explanatory only and are not restrictive of the invention.

## BRIEF DESCRIPTION OF THE DRAWINGS

The accompanying drawings, which are incorporated in and constitute a part of this specification, illustrate embodiments consistent with the present invention and, together with the description, serve to explain the principles of the present invention.

Fig. 1 is a flowchart of a method for predicting fiber orientation according to an exemplary embodiment.

Fig. 2 is a diagram showing a positional relationship between fibers after spinning according to an exemplary embodiment.

Fig. 3 is a diagram showing a positional relationship between fibers after the fiber composite material is subject to a forming process according to an exemplary embodiment.

Fig. 4 is a schematic diagram of a change value of fiber property of the fiber composite material according to an exemplary embodiment.

Fig. 5 is a schematic diagram of an initial structure of fiber according to an exemplary embodiment.

Fig. 6 is a schematic diagram of a fiber orientation prediction according to an exemplary embodiment.

Fig. 7 is a schematic diagram of a limit structure of fiber orientation according to an exemplary embodiment.

Fig. 8 is a block diagram of a device for predicting fiber orientation according to an exemplary embodiment.

Fig. 9 is a block diagram of a second obtaining module in a device for predicting fiber orientation according to an exemplary embodiment.

Fig. 10 is a block diagram of a device for predicting fiber orientation according to an exemplary embodiment.

Fig. 11 is a block diagram of a first obtaining module in a device for predicting fiber orientation according to an exemplary embodiment.

## DETAILED DESCRIPTION

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the invention. Instead, they are merely examples of apparatuses and methods consistent with aspects related to the invention as recited in the appended claims.

In order to solve the above technical problem, the present invention provides a method for predicting fiber orientation, by which fiber orientation of a three-dimensionally woven long fiber composite material can be predicted.

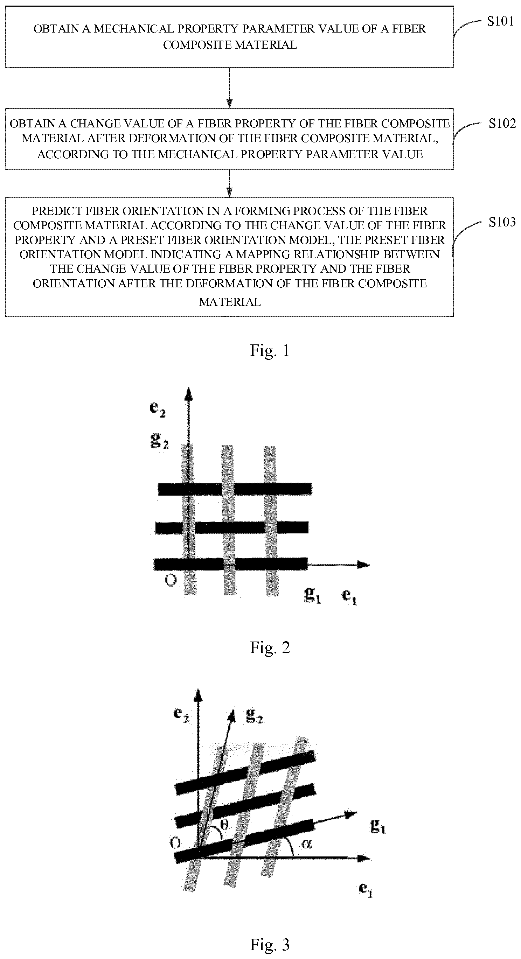

Fig. 1 is a flowchart of a method for predicting fiber orientation according to an exemplary embodiment. As shown in Fig. 1, the method includes the following steps S101-S103:

In step S101, a mechanical property parameter value of a fiber composite material is obtained.

The mechanical property parameter value of the fiber composite material can be obtained by using preset simulation software.

For example, the mechanical property parameter value of the composite material can be obtained by using the finite element software Digimat.

Of course, before obtaining the mechanical property parameter value of the fiber composite material by using the preset simulation software, it is also possible to determine whether the mechanical property parameter value of the fiber composite material can be obtained through experiments. If experiment conditions are not met, the mechanical property parameter value of the fiber composite material is obtained by using preset simulation software.

In step S102, a change value of a fiber property of the fiber composite material after deformation of the fiber composite material is obtained, according to the mechanical property parameter value.

In step S103, fiber orientation in a forming process of the fiber composite material is predicted according to the change value of the fiber property and a preset fiber orientation model. The preset fiber orientation model indicates a mapping relationship between the change value of fiber property and the fiber orientation after the deformation of the fiber composite material.

The fiber orientation refers to directions of fibers in a woven structure, which are generally perpendicular to each other after completion of weaving (as shown in Fig. 2); and the directions of the fibers may be changed with the process (as shown in Fig. 3). Therefore, performance of the composite material is closely related to a distribution state of interior fiber orientation, and thus calculation, analysis and prediction of the fiber orientation are of great significance.

Referring to Figs. 2 and 3, predicting the fiber orientation in a forming process of fiber composite material is to predict $\theta$ in Fig. 3. The present invention introduces a preset fiber orientation model for indicating a mapping relationship between a change value of a fiber property and fiber orientation after deformation of a fiber composite material, so that fiber orientation in a forming process of the fiber composite material can be predicted from the fiber change value as obtained.

It should be noted that the fiber composite material in the present invention includes a long fiber composite material; and the long fiber composite material includes a three-dimensionally woven long fiber composite material.

The technical solution provided in embodiment of the present invention may have the following beneficial effects: obtaining a mechanical property parameter value of a fiber composite material; obtaining a change value of a fiber property of the fiber composite material after deformation of the fiber composite material, according to the mechanical property parameter value; and predicting fiber orientation in a forming process of the fiber composite material according to the change value of the fiber property and a preset fiber orientation model, the preset fiber orientation model indicating a mapping relationship between the change value of the fiber property and the fiber orientation after the deformation of the fiber composite material. The present invention introduces a preset fiber orientation model for indicating a mapping relationship between the change value of the fiber property and the fiber orientation after deformation of the fiber composite material, so that fiber orientation in a forming process of the fiber composite material can be predicted from the fiber change value as obtained.

For example, the change value of the fiber property includes a change value of a fiber in a preset Cartesian coordinate system and a change value of a fiber in a relative coordinate system, the relative coordinate system being obtained according to a warp direction and a weft direction of the fiber composite material;

the change value of the fiber in the preset Cartesian coordinate system includes: a length change value of the fiber along respective coordinate axes in the preset Cartesian coordinate system, and a change value of an angle between a warp fiber and a weft fiber in the composite material in the preset Cartesian coordinate system; and

the change value of the fiber in the relative coordinate system includes: a length change value of the fiber along respective coordinate axes in the relative coordinate system, and a change value of an angle between a warp fiber and a weft fiber in the composite material in the relative coordinate system.

As shown in Fig. 2 and Fig. 3, Fig. 2 is a diagram showing a positional relationship between fibers after spinning according to an exemplary embodiment, in which fibers are typically perpendicular to each other, and Fig. 3 is a diagram showing a positional relationship between fibers after a fiber composite is subject to a forming process according to an exemplary embodiment. A coordinate system composed of coordinate axes $e_1$ and $e_2$ is a preset Cartesian coordinate system, and a coordinate system composed of coordinate axes $g_1$ and $g_2$ is a relative coordinate system.

In an embodiment, obtaining a change value of a fiber property of the fiber composite material after deformation of the fiber composite material includes the following substep:

obtaining a length change value of a fiber in the fiber composite material along respective coordinate axes in a preset Cartesian coordinate system and a change value of an angle between a warp fiber and a weft fiber in the fiber composite material in the preset Cartesian coordinate system, after the deformation of the fiber composite material.

The above substep may include the following substeps A1-A3:

A1: obtaining a first length value of the fiber along respective coordinate axes in the preset Cartesian coordinate system and a first angle value of an angle between the warp fiber and the weft fiber in the composite material in the preset Cartesian coordinate system, before deformation of the fiber;

A2: obtaining a second length value of the fiber along respective coordinate axes in the preset Cartesian coordinate system and a second angle value of an angle between the warp fiber and the weft fiber in the composite material in the preset Cartesian coordinate system, after the deformation of the fiber; and

A3: obtaining a length change value of the fiber along respective coordinate axes in the preset Cartesian coordinate system according to the second length value and the first length value, and obtaining a change value of the angle between the warp fiber and the weft fiber in the fiber composite material in the preset Cartesian coordinate system according to the second angle value and the first angle value.

After obtaining the first length value of the fiber along respective coordinate axes in the preset Cartesian coordinate system before deformation of the fiber and the second length value of the fiber along respective coordinate axes in the preset Cartesian coordinate system after the deformation of the fiber, the length change value of the fiber along respective coordinate axes in the preset Cartesian coordinate system can be obtained by subtracting the first length value from the second length value.

Similarly, after obtaining the first angle value of an angle between the warp fiber and the weft fiber in the composite material in the preset Cartesian coordinate system before deformation of the fiber and the second angle value of an angle between the warp fiber and the weft fiber in the composite material in the preset Cartesian coordinate system after the deformation of the fiber, the change value of the angle between the warp fiber and the weft fiber in the fiber composite material in the preset Cartesian coordinate system can be obtained by subtracting the first angle value from the second angle value.

The above steps can be implemented by simulation software, and the invention does not limit the type of the simulation software.

In an embodiment, obtaining a change value of a fiber property of the fiber composite material after the deformation of the fiber composite material includes the following substep:

obtaining a length change value of a fiber in the fiber composite material along respective coordinate axes in the relative coordinate system and a change value of an angle between a warp fiber and a weft fiber in the fiber composite material in the relative coordinate system, after the deformation of the fiber composite material.

The above substep may include the following steps B1-B3:

B1: obtaining a third length value of the fiber along respective coordinate axes in the relative coordinate system and a third angle value of an angle between the warp fiber and the weft fiber in the composite material in the relative coordinate system, before deformation of the fiber;

B2: obtaining a fourth length value of the fiber along respective coordinate axes in the relative coordinate system and a fourth angle value of an angle between the warp fiber and the weft fiber in the composite material in the relative coordinate system, after the deformation of the fiber; and

B3: obtaining a length change value of the fiber along respective coordinate axes in the relative coordinate system according to the fourth length value and the third length value, and obtaining a change value of the angle between the warp fiber and the weft fiber in the composite material in the relative coordinate system according to the fourth angle value and the third angle value.

After obtaining the third length value of the fiber along respective coordinate axes in the relative coordinate system before deformation of the fiber and the fourth length value of the fiber along respective coordinate axes in the relative coordinate system after the deformation of the fiber, the length change value of the fiber along respective coordinate axes in the relative coordinate system can be obtained by subtracting the third length value from the fourth length value.

Similarly, after obtaining the third angle value of an angle between the warp fiber and the weft fiber in the composite material in the relative coordinate system before deformation of the fiber and the fourth angle value of an angle between the warp fiber and the weft fiber in the composite material in the relative coordinate system after the deformation of the fiber, the change value of the angle between the warp fiber and the weft fiber of the fiber composite material in the relative coordinate system can be obtained by subtracting the third angle value from the fourth angle value.

The above steps can be implemented by simulation software, and the invention does not limit the type of the simulation software.

In an embodiment, prior to said predicting fiber orientation in the fiber composite material according to the change value of the fiber property and a preset fiber orientation model, the method further includes the following substeps C1-C3:

C1: obtaining a Cartesian component of stress-strain tensor according to a change value of the fiber in a preset Cartesian coordinate system. The change value of the fiber in the preset Cartesian coordinate system includes: a length change value of the fiber along respective coordinate axes in the predetermined Cartesian coordinate system, and a change value of an angle between a warp fiber and a weft fiber in the composite material in the predetermined Cartesian coordinate system.

For example, the Cartesian component of the stress-strain tensor may be expressed as:

$$\left\{ \begin{array}{c} d\varepsilon_{11} \\ d\varepsilon_{22} \\ d\gamma_{12} \end{array} \right\}$$

where $d\varepsilon_{11}$ represents a length change value of the fiber along a first coordinate axis in the preset Cartesian coordinate system; $d\varepsilon_{22}$ represents a length change value of the fiber along a second coordinate axis in the preset Cartesian coordinate system; and $d\gamma_{12}$ represents a change value of an angle between a warp fiber and a weft fiber in the composite material in the preset Cartesian coordinate system.

Taking Fig. 2 and Fig. 3 as an example, $d\varepsilon_{11}$ represents a length change value of the fiber along a coordinate axis $e_1$ in a coordinate system composed of the coordinate axis $e_1$ and a coordinate axis $e_2$; $d\varepsilon_{22}$ represents a length change value of the fiber along the coordinate axis $e_2$ in the coordinate system composed of the coordinate axis $e_1$ and the coordinate axis $e_2$; and $d\gamma_{12}$ represents a change value of the angle between the warp fiber and the weft fiber in the composite material in the coordinate system composed of the coordinate axis $e_1$ and the coordinate axis $e_2$.

C2: obtaining a relative coordinate component of the stress-strain tensor according to a change value of the fiber in a relative coordinate system. The change value of the fiber in the relative coordinate system including: a length change value of the fiber along respective coordinate axes in the relative coordinate system, and a change value of an angle between a warp fiber and a weft fiber in the composite material in the relative coordinate system.

For example, the relative coordinate component of the stress-strain tensor can be expressed as:

$$\left\{ \begin{array}{c} d\bar{\varepsilon}_{11} \\ d\bar{\varepsilon}_{22} \\ d\bar{\gamma}_{12} \end{array} \right\}$$

where $d\bar{\varepsilon}_{11}$ represents a length change value of the fiber along a first coordinate axis in the relative coordinate system; $d\bar{\varepsilon}_{22}$ represents a length change value of the fiber along a second coordinate axis in the relative coordinate system; and $d\bar{\gamma}_{12}$ represents a change value of an angle between a warp fiber and a weft fiber in the composite material in the relative coordinate system.

Taking Fig. 2 and Fig. 3 as an example, $d\bar{\varepsilon}_{11}$ represents a length change value of the fiber along a coordinate axis $g_1$ in a coordinate system composed of the coordinate axis $g_1$ and a coordinate axis $g_2$; $d\bar{\varepsilon}_{22}$ represents a length change value of the fiber along the coordinate axis $g_2$ in the coordinate system composed of the coordinate axis $g_1$ and the coordinate axis $g_2$; and $d\bar{\gamma}_{12}$ represents a change value of the angle between the warp fiber and the weft fiber in the composite material in the coordinate system composed of the coordinate axis $g_1$ and the coordinate axis $g_2$.

C3: obtaining the preset fiber orientation model according to a relationship among a Cartesian component of the stress-strain tensor, a relative coordinate component of the stress-strain tensor, and fiber orientation.

Since the three-dimensional woven long fiber composite material is a typical anisotropic material, non-uniformity and anisotropy of the material are the main difficulties. In this embodiment, in order to select a suitable preset fiber orientation model, a plane coordinate system (i.e., the relative coordinate system described above) that is consistent with the weft and warp of the woven fabric can be embedded in a shell element model. The relative coordinate components (i.e., an inverse stress component and a covariation strain component) of the stress-strain tensor in the constitutive relationship are introduced into the relative coordinate system described above. The preset fiber orientation model of the fiber composite material is derived by the transformation between the relative coordinate component of the stress-strain tensor and the Cartesian component of the stress-strain tensor.

Further, since stretching and shearing can be decoupled in the above constitutive relationship, the preset fiber orientation model can be obtained based on the above-described constitutive relationship.

In an embodiment, the relationship among the Cartesian component of the stress-strain tensor, the relative coordinate component of the stress-strain tensor and the fiber orientation includes:

the relative coordinate component of the stress-strain tensor is a product of the Cartesian component of the stress-strain tensor, and a preset trigonometric function value corresponding to an angular value of the fiber orientation.

For example, the relative coordinate component of the stress-strain tensor being a product of the Cartesian component of the stress-strain tensor, and a preset trigonometric function value corresponding to an angular value of the fiber orientation can be expressed as:

$$\left\{ \begin{array}{c} d\bar{\varepsilon}_{11} \\ d\bar{\varepsilon}_{22} \\ d\bar{\gamma}_{12} \end{array} \right\} = \left[ \begin{array}{ccc} \cos^2 \alpha & \sin^2 \alpha & \sin \alpha \cos \alpha \\ \cos^2(\alpha + \theta) & \sin^2(\alpha + \theta) & \sin(\alpha + \theta)\cos(\alpha + \theta) \\ 2\cos \alpha \cos(\alpha + \theta) & 2\sin \alpha \sin(\alpha + \theta) & \sin(2\alpha + \theta) \end{array} \right] \left\{ \begin{array}{c} d\varepsilon_{11} \\ d\varepsilon_{22} \\ d\gamma_{12} \end{array} \right\}$$

where the $\alpha$ represents an angle change value of the angle between the fiber and the first coordinate axis in the preset Cartesian coordinate system; and the $\theta$ represents an angle value of the fiber orientation.

Taking Fig. 2 and Fig. 3 as an example, the $\alpha$ represents an angle change value of the angel between the fiber and the coordinate axis $e_1$ in the coordinate system composed of the coordinate axis $e_1$ and the coordinate axis $e_2$; and the $\theta$ represents an angle value of the fiber orientation.

In practical application, the finite element software Abaqus can be used to predict fiber orientation in a forming process of fiber composite material.

Specifically, using Abaqus' VUMAT user-defined subroutine function, a user can input the above fiber orientation model via VUMAT.

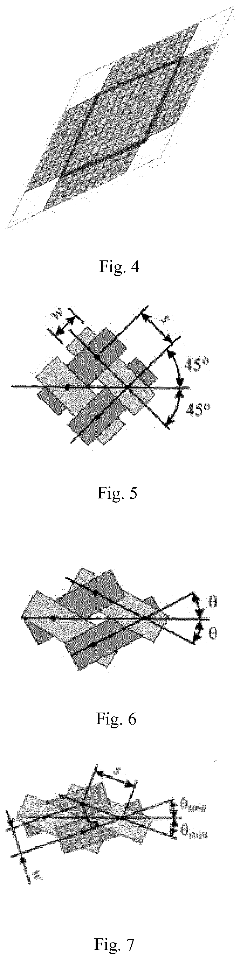

Since the Abaqus software is able to calculate the change value of the fiber property of the fiber composite material (as shown in Fig. 4), the Abaqus software can predict fiber orientation in the forming process of the fiber composite material by calculating the change value of the fiber property of the fiber composite material and by using the input fiber orientation model.

In this case, the distribution result of the fiber orientation is output as shown in Figs. 5-7. Fig. 5 is a schematic diagram of an initial structure of fiber according to an exemplary embodiment, and Fig. 6 is a schematic diagram of a fiber orientation prediction according to an exemplary embodiment. Further, Fig. 7 is a schematic diagram of a limit structure of fiber orientation according to an exemplary embodiment. That is, the angle value $\theta$ in the fiber orientation has a minimum value $\theta_{min}$ when the fiber composite material deforms, where w is the width of the fiber and s is the distance between the fibers.

The following is a device embodiment of the present invention, which may be used to implement the method embodiments of the present invention.

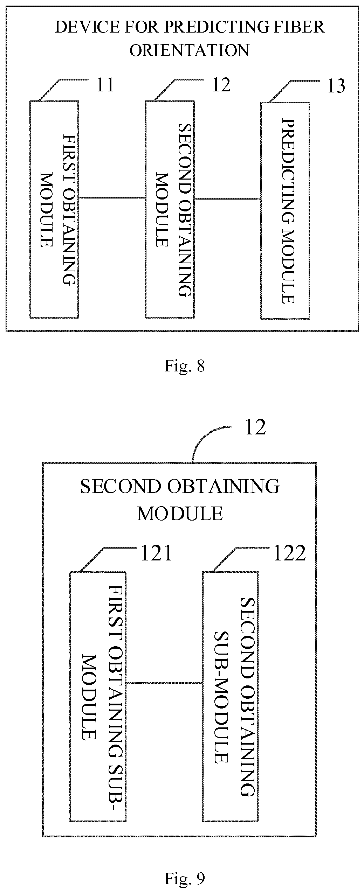

Fig. 8 is a block diagram of a device for predicting fiber orientation according to an exemplary embodiment. As shown in Fig. 8, the device for predicting fiber orientation includes:

a first obtaining module 11, configured to a mechanical property parameter value of a fiber composite material;

a second obtaining module 12, configured to obtain a change value of a fiber property of the fiber composite material after deformation of the fiber composite material, according to the mechanical property parameter value obtained by the first obtaining module 11; and

a predicting module 13, configured to predict fiber orientation in a forming process of the fiber composite material according to the change value of the fiber property obtained by the second obtaining module 12 and a preset fiber orientation model, the preset fiber orientation model indicating a mapping relationship between the change value of the fiber property and the fiber orientation after the deformation of the fiber composite material.

In an embodiment, as shown in Fig. 9, the second obtaining module 12 includes a first obtaining sub-module 121 and a second obtaining sub-module 122,

the first obtaining sub-module 121 is configured to obtain a length change value of a fiber in the fiber composite material along respective coordinate axes in a predetermined Cartesian coordinate system and a change value of an angle between a warp fiber and a weft fiber in the fiber composite material in the predetermined Cartesian coordinate system, after the deformation of the fiber composite material; and

the second obtaining sub-module 122 is configured to obtain a length change value of a fiber in the fiber composite material along respective coordinate axes in a relative coordinate system and a change value of an angle between a warp fiber and a weft fiber in the fiber composite material in the relative coordinate system after the deformation of the fiber composite material.

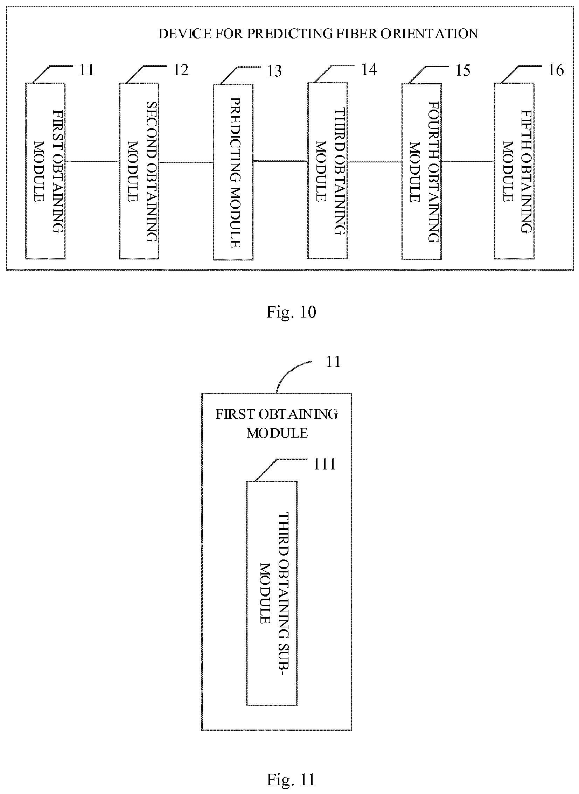

In an embodiment, as shown in Fig. 10, the device further includes: a third obtaining module 14, a fourth obtaining module 15, and a fifth obtaining module 16,

the third obtaining module 14 is configured to obtain a Cartesian component of stress-strain tensor according to a change value of the fiber in a preset Cartesian coordinate system, the change value of the fiber in the preset Cartesian coordinate system including: a length change value of the fiber along respective coordinate axes in the predetermined Cartesian coordinate system, and a change value of an angle between a warp fiber and a weft fiber in the composite material in the predetermined Cartesian coordinate system;

the fourth obtaining module 15 is configured to obtain a relative coordinate component of the stress-strain tensor according to a change value of the fiber in a relative coordinate system, the change value of the fiber in the relative coordinate system including: a length change value of the fiber along respective coordinate axes in the relative coordinate system, and a change value of an angle between a warp fiber and a weft fiber in the composite material in the relative coordinate system; and

the fifth obtaining module 16 is configured to obtain the preset fiber orientation model according to a relationship among a Cartesian component of the stress-strain tensor obtained by the third obtaining module 14, a relative coordinate component of the stress-strain tensor obtained by the fourth obtaining module 15, and fiber orientation.

In an embodiment, the Cartesian component of the stress-strain tensor is expressed as:

$$\left\{ \begin{array}{c} d\varepsilon_{11} \\ d\varepsilon_{22} \\ d\gamma_{12} \end{array} \right\}$$

where $d\varepsilon_{11}$ represents a length change value of the fiber along a first coordinate axis in the preset Cartesian coordinate system; $d\varepsilon_{22}$ represents a length change value of the fiber along a second coordinate axis in the preset Cartesian coordinate system; and $d\gamma_{12}$ represents a change value of an angle between a warp fiber and a weft fiber in the composite material in the preset Cartesian coordinate system.

In an embodiment, the relative coordinate component of the stress-strain tensor is expressed as:

$$\left\{ \begin{array}{c} d\bar{\varepsilon}_{11} \\ d\bar{\varepsilon}_{22} \\ d\bar{\gamma}_{12} \end{array} \right\}$$

where $d\bar{\varepsilon}_{11}$ represents a length change value of the fiber along a first coordinate axis in the relative coordinate system; $d\bar{\varepsilon}_{22}$ represents a length change value of the fiber along a second coordinate axis in the relative coordinate system; and $d\bar{\gamma}_{12}$ represents a change value of an angle between a warp fiber and a weft fiber in the relative coordinate system.

In an embodiment, the relationship among the Cartesian component of the stress-strain tensor, the relative coordinate component of the stress-strain tensor and the fiber orientation includes:

the relative coordinate component of the stress-strain tensor is a product of the Cartesian component of the stress-strain tensor, and a preset trigonometric function value corresponding to an angular value of the fiber orientation.

In an embodiment, said the relative coordinate component of the stress-strain tensor being a product of the Cartesian component of the stress-strain tensor, and a preset trigonometric function value corresponding to an angular value of the fiber orientation includes:

$$\left\{ \begin{array}{c} d\bar{\varepsilon}_{11} \\ d\bar{\varepsilon}_{22} \\ d\bar{\gamma}_{12} \end{array} \right\} = \left[ \begin{array}{ccc} \cos^2\alpha & \sin^2\alpha & \sin\alpha\cos\alpha \\ \cos^2(\alpha+\theta) & \sin^2(\alpha+\theta) & \sin(\alpha+\theta)\cos(\alpha+\theta) \\ 2\cos\alpha\cos(\alpha+\theta) & 2\sin\alpha\sin(\alpha+\theta) & \sin(2\alpha+\theta) \end{array} \right] \left\{ \begin{array}{c} d\varepsilon_{11} \\ d\varepsilon_{22} \\ d\gamma_{12} \end{array} \right\}$$

where the $\alpha$ represents an angle change value of the angle between the fiber and the first coordinate axis in the preset Cartesian coordinate system; the $\theta$ represents an angle value of the fiber orientation.

In an embodiment, as shown in Fig. 11, the first obtaining module 11 includes a third obtaining sub-module 111; the third obtaining sub-module 111 is configured to obtain the mechanical property parameter value of the fiber composite material by using preset simulation software.

For the device in the above embodiment, the specific manners in which various modules operate have been described in detail in the embodiment relating to the above method, and therefore will not be repeated herein.

Other embodiments of the invention will be apparent to those skilled in the art from consideration of the specification and practice of the invention disclosed here. This invention is intended to cover any variations, uses, or adaptations of the invention following the general principles thereof and including such departures from the present invention as come within known or customary practice in the art. It is intended that the specification and examples be considered as exemplary only, with a true scope of the invention being indicated by the following claims.

It will be appreciated that the present invention is not limited to the exact construction that has been described above and illustrated in the accompanying drawings, and that various modifications and changes may be made without departing from the scope thereof. It is intended that the scope of the invention only be limited by the appended claims.

**Claims**

1. A method for predicting fiber orientation, comprising:

   obtaining (S101) a mechanical property parameter value of a fiber composite material;
   obtaining (S102) a change value of a fiber property of the fiber composite material after deformation of the fiber composite material, according to the mechanical property parameter value; and
   predicting (S103) fiber orientation in a forming process of the fiber composite material according to the change value of the fiber property and a preset fiber orientation model, wherein the preset fiber orientation model indicates a mapping relationship between the change value of the fiber property and the fiber orientation after the deformation of the fiber composite material.

2. The method according to claim 1, wherein said obtaining (S102) a change value of a fiber property of the fiber composite material after deformation of the fiber composite material, according to the mechanical property parameter value comprises:

   obtaining a length change value of a fiber in the fiber composite material along respective coordinate axes in a predetermined Cartesian coordinate system and a change value of an angle between a warp fiber and a weft fiber in the fiber composite material in the predetermined Cartesian coordinate system, after the deformation of the fiber composite material; and
   obtaining a length change value of a fiber in the fiber composite material along respective coordinate axes in a relative coordinate system and a change value of an angle between a warp fiber and a weft fiber in the fiber composite material in the relative coordinate system, after the deformation of the fiber composite material.

3. The method according to claim 2, wherein prior to said predicting (S103) fiber orientation in the fiber composite material according to the change value of the fiber property and a preset fiber orientation model, the method further comprises:

   obtaining a Cartesian component of stress-strain tensor according to a change value of the fiber in a preset Cartesian coordinate system, wherein the change value of the fiber in the preset Cartesian coordinate system comprises: a length change value of the fiber along respective coordinate axes in the predetermined Cartesian coordinate system, and a change value of an angle between a warp fiber and a weft fiber in the composite material in the predetermined Cartesian coordinate system;
   obtaining a relative coordinate component of the stress-strain tensor according to a change value of the fiber in a relative coordinate system, wherein the change value of the fiber in the relative coordinate system comprises: a length change value of the fiber along respective coordinate axes in the relative coordinate system, and a change value of an angle between a warp fiber and a weft fiber in the composite material in the relative coordinate system; and
   obtaining the preset fiber orientation model according to a relationship among a Cartesian component of the stress-strain tensor, a relative coordinate component of the stress-strain tensor, and fiber orientation.

4. The method according to claim 3, wherein the Cartesian component of the stress-strain tensor is expressed as:

$$\left\{\begin{array}{c} d\varepsilon_{11} \\ d\varepsilon_{22} \\ d\gamma_{12} \end{array}\right\}$$

where $d\varepsilon_{11}$ represents a length change value of the fiber along a first coordinate axis in the preset Cartesian coordinate system; $d\varepsilon_{22}$ represents a length change value of the fiber along a second coordinate axis in the preset Cartesian coordinate system; and $d\gamma_{12}$ represents a change value of an angle between a warp fiber and a weft fiber in the composite material in the preset Cartesian coordinate system.

5. The method according to claim 4, wherein the relative coordinate component of the stress-strain tensor is expressed as:

$$\left\{\begin{array}{c} d\bar{\varepsilon}_{11} \\ d\bar{\varepsilon}_{22} \\ d\bar{\gamma}_{12} \end{array}\right\}$$

where $d\bar{\varepsilon}_{11}$ represents a length change value of the fiber along a first coordinate axis in the relative coordinate system; $d\bar{\varepsilon}_{22}$ represents a length change value of the fiber along a second coordinate axis in the relative coordinate system; and $d\bar{\gamma}_{12}$ represents a change value of an angle between a warp fiber and a weft fiber in the composite material in the relative coordinate system.

6. The method according to claim 5, wherein the relationship among the Cartesian component of the stress-strain tensor, the relative coordinate component of the stress-strain tensor and the fiber orientation comprises:

the relative coordinate component of the stress-strain tensor is a product of the Cartesian component of the stress-strain tensor, and a preset trigonometric function value corresponding to an angular value of the fiber orientation.

7. The method according to claim 6, wherein said the relative coordinate component of the stress-strain tensor being a product of the Cartesian component of the stress-strain tensor, and a preset trigonometric function value corresponding to an angular value of the fiber orientation comprises:

$$\left\{\begin{array}{c} d\bar{\varepsilon}_{11} \\ d\bar{\varepsilon}_{22} \\ d\bar{\gamma}_{12} \end{array}\right\} = \left[\begin{array}{ccc} \cos^2\alpha & \sin^2\alpha & \sin\alpha\cos\alpha \\ \cos^2(\alpha+\theta) & \sin^2(\alpha+\theta) & \sin(\alpha+\theta)\cos(\alpha+\theta) \\ 2\cos\alpha\cos(\alpha+\theta) & 2\sin\alpha\sin(\alpha+\theta) & \sin(2\alpha+\theta) \end{array}\right] \left\{\begin{array}{c} d\varepsilon_{11} \\ d\varepsilon_{22} \\ d\gamma_{12} \end{array}\right\}$$

where the $\alpha$ represents an angle change value of the angle between the fiber and the first coordinate axis in the preset Cartesian coordinate system; and the $\theta$ represents an angle value of the fiber orientation.

8. The method according to claim 1 wherein said obtaining (S101) a mechanical property parameter value of a fiber composite material comprises:

obtaining the mechanical property parameter value of the fiber composite material by using preset simulation software.

9. A device for predicting fiber orientation, comprising:

a first obtaining module (11), configured to obtain a mechanical property parameter value of a fiber composite material;
a second obtaining module (12), configured to obtain a change value of a fiber property of the fiber composite material after deformation of the fiber composite material, according to the mechanical property parameter value obtained by the first obtaining module (11); and

a predicting module (13), configured to predict fiber orientation in a forming process of the fiber composite material according to the change value of the fiber property obtained by the second obtaining module (12) and a preset fiber orientation model, wherein the preset fiber orientation model indicates a mapping relationship between the change value of the fiber property and the fiber orientation after the deformation of the fiber composite material.

10. The device according to claim 9, wherein the second obtaining module (12) comprises a first obtaining sub-module (121) and a second obtaining sub-module (122),

the first obtaining sub-module (121) is configured to obtain a length change value of a fiber in the fiber composite material along respective coordinate axes in a predetermined Cartesian coordinate system and a change value of an angle between a warp fiber and a weft fiber in the fiber composite material in the predetermined Cartesian coordinate system, after the deformation of the fiber composite material; and

the second obtaining sub-module (122) is configured to obtain a length change value of a fiber in the fiber composite material along respective coordinate axes in a relative coordinate system and a change value of an angle between a warp fiber and a weft fiber in the fiber composite material in the relative coordinate system, after the deformation of the fiber composite material.

11. The device according to claim 10, wherein the device further comprises a third obtaining module (14), a fourth obtaining module (15), and a fifth obtaining module (16),

the third obtaining module (14) is configured to obtain a Cartesian component of stress-strain tensor according to a change value of the fiber in a preset Cartesian coordinate system, wherein the change value of the fiber in the preset Cartesian coordinate system comprises: a length change value of the fiber along respective coordinate axes in the predetermined Cartesian coordinate system, and a change value of an angle between a warp fiber and a weft fiber in the composite material in the predetermined Cartesian coordinate system;

the fourth obtaining module (15) is configured to obtain a relative coordinate component of the stress-strain tensor according to a change value of the fiber in a relative coordinate system, wherein the change value of the fiber in the relative coordinate system comprises: a length change value of the fiber along respective coordinate axes in the relative coordinate system, and a change value of an angle between a warp fiber and a weft fiber in the composite material in the relative coordinate system; and

the fifth obtaining module (16) is configured to obtain the preset fiber orientation model according to a relationship among a Cartesian component of the stress-strain tensor obtained by the third obtaining module (14), a relative coordinate component of the stress-strain tensor obtained by the fourth obtaining module (15), and fiber orientation.

12. The device according to claim 11, wherein the Cartesian component of the stress-strain tensor is expressed as:

$$\left\{ \begin{array}{c} d\varepsilon_{11} \\ d\varepsilon_{22} \\ d\gamma_{12} \end{array} \right\}$$

where $d\varepsilon_{11}$ represents a length change value of the fiber along a first coordinate axis in the preset Cartesian coordinate system; $d\varepsilon_{22}$ represents a length change value of the fiber along a second coordinate axis in the preset Cartesian coordinate system; and $d\gamma_{12}$ represents a change value of an angle between a warp fiber and a weft fiber in the composite material in the preset Cartesian coordinate system.

13. The device according to claim 12, wherein the relative coordinate component of the stress-strain tensor is expressed as:

$$\left\{ \begin{array}{c} d\bar{\varepsilon}_{11} \\ d\bar{\varepsilon}_{22} \\ d\bar{\gamma}_{12} \end{array} \right\}$$

where $d\bar{\varepsilon}_{11}$ represents a length change value of the fiber along a first coordinate axis in the relative coordinate

system; $d\overline{\varepsilon}_{22}$ represents a length change value of the fiber along a second coordinate axis in the relative coordinate system; and $d\overline{\gamma}_{12}$ represents a change value of an angle between a warp fiber and a weft fiber in the relative coordinate system.

**14.** The device according to claim 13, wherein the relationship among the Cartesian component of the stress-strain tensor, the relative coordinate component of the stress-strain tensor and the fiber orientation comprises:

the relative coordinate component of the stress-strain tensor is a product of the Cartesian component of the stress-strain tensor, and a preset trigonometric function value corresponding to an angular value of the fiber orientation.

**15.** The device according to claim 14, wherein said the relative coordinate component of the stress-strain tensor being a product of the Cartesian component of the stress-strain tensor, and a preset trigonometric function value corresponding to an angular value of the fiber orientation comprises:

$$\begin{Bmatrix} d\overline{\varepsilon}_{11} \\ d\overline{\varepsilon}_{22} \\ d\overline{\gamma}_{12} \end{Bmatrix} = \begin{bmatrix} \cos^2 \alpha & \sin^2 \alpha & \sin \alpha \cos \alpha \\ \cos^2(\alpha + \theta) & \sin^2(\alpha + \theta) & \sin(\alpha + \theta)\cos(\alpha + \theta) \\ 2\cos \alpha \cos(\alpha + \theta) & 2\sin \alpha \sin(\alpha + \theta) & \sin(2\alpha + \theta) \end{bmatrix} \begin{Bmatrix} d\varepsilon_{11} \\ d\varepsilon_{22} \\ d\gamma_{12} \end{Bmatrix}$$

where the $\alpha$ represents an angle change value of the angle between the fiber and the first coordinate axis in the preset Cartesian coordinate system; the $\theta$ represents an angle value of the fiber orientation.

| OBTAIN A MECHANICAL PROPERTY PARAMETER VALUE OF A FIBER COMPOSITE MATERIAL | S101 |

| OBTAIN A CHANGE VALUE OF A FIBER PROPERTY OF THE FIBER COMPOSITE MATERIAL AFTER DEFORMATION OF THE FIBER COMPOSITE MATERIAL, ACCORDING TO THE MECHANICAL PROPERTY PARAMETER VALUE | S102 |

| PREDICT FIBER ORIENTATION IN A FORMING PROCESS OF THE FIBER COMPOSITE MATERIAL ACCORDING TO THE CHANGE VALUE OF THE FIBER PROPERTY AND A PRESET FIBER ORIENTATION MODEL, THE PRESET FIBER ORIENTATION MODEL INDICATING A MAPPING RELATIONSHIP BETWEEN THE CHANGE VALUE OF THE FIBER PROPERTY AND THE FIBER ORIENTATION AFTER THE DEFORMATION OF THE FIBER COMPOSITE MATERIAL | S103 |

Fig. 1

Fig. 2

Fig. 3

Fig. 4

Fig. 5

Fig. 6

Fig. 7

DEVICE FOR PREDICTING FIBER
ORIENTATION

11      12      13

FIRST OBTAINING
MODULE

SECOND OBTAINING
MODULE

PREDICTING MODULE

Fig. 8

12

SECOND OBTAINING
MODULE

121      122

FIRST OBTAINING SUB-
MODULE

SECOND OBTAINING
SUB-MODULE

Fig. 9

DEVICE FOR PREDICTING FIBER ORIENTATION

11     12     13     14     15     16

FIRST OBTAINING MODULE

SECOND OBTAINING MODULE

PREDICTING MODULE

THIRD OBTAINING MODULE

FOURTH OBTAINING MODULE

FIFTH OBTAINING MODULE

Fig. 10

11

FIRST OBTAINING MODULE

111

THIRD OBTAINING SUB-MODULE

Fig. 11

Europäisches
Patentamt

European
Patent Office

Office européen
des brevets

# EUROPEAN SEARCH REPORT

Application Number

EP 18 19 6558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XUE P ET AL: "A non-orthogonal constitutive model for characterizing woven composites", COMPOSITES PART A: APPLIED SCIENCE AND MANUFACTURING, ELSEVIER, AMSTERDAM, NL, vol. 34, no. 2, 1 February 2003 (2003-02-01), pages 183-193, XP004412441, ISSN: 1359-835X, DOI: 10.1016/S1359-835X(02)00052-0 * abstract * * page 183, column 2 - page 184, column 1, paragraph 2 * * page 185, column 2, paragraph 4 - page 188, column 1 * | 1-15 | INV. G06F17/50 |
| X | Y Baillargeon ET AL: "Prediction of fiber orientation and microstructure of woven fabric composites after forming", Composite Structrues Volume 52 Issues 3-4, 1 May 2001 (2001-05-01), pages 475-481, XP055581382, Retrieved from the Internet: URL:https://ac.els-cdn.com/S026382230100037X/1-s2.0-S026382230100037X-main.pdf?_tid=4fde634e-c88c-433b-8ad2-c7e271c7ba68&acdnat=1555402194_acffadaab01741b7c715deb5f58e838d [retrieved on 2019-04-16] * abstract * * page 475 - page 478 * | 1-15 | |

TECHNICAL FIELDS SEARCHED (IPC)

G06F

-/--

The present search report has been drawn up for all claims

| Place of search | Date of completion of the search | Examiner |
|---|---|---|
| Munich | 17 April 2019 | Radev, Boyan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

**EUROPEAN SEARCH REPORT**

Application Number

EP 18 19 6558

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIONGQI PENG ET AL: "Textile composite double dome stamping simulation using a non-orthogonal constitutive model", COMPOSITES SCIENCE AND TECHNOLOGY, ELSEVIER, AMSTERDAM, NL, vol. 71, no. 8, 17 March 2011 (2011-03-17), pages 1075-1081, XP028212298, ISSN: 0266-3538, DOI: 10.1016/J.COMPSCITECH.2011.03.010 [retrieved on 2011-03-24] * abstract * * page 1076 - page 1079 * | 1-15 | |
| A | XIONGQI PENG ET AL: "Validation of a non-orthogonal constitutive model for woven composite fabrics via hemispherical stamping simulation", COMPOSITES PART A: APPLIED SCIENCE AND MANUFACTURING, ELSEVIER, AMSTERDAM, NL, vol. 42, no. 4, 18 December 2010 (2010-12-18), pages 400-407, XP028357723, ISSN: 1359-835X, DOI: 10.1016/J.COMPOSITESA.2010.12.014 [retrieved on 2010-12-24] * abstract * * page 402 * | 1-15 | TECHNICAL FIELDS SEARCHED (IPC) |

-/--

The present search report has been drawn up for all claims

| Place of search | Date of completion of the search | Examiner |
|---|---|---|
| Munich | 17 April 2019 | Radev, Boyan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 19 6558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BICKERTON S ET AL: "Investigation of draping and its effects on the mold filling process during manufacturing of a compound curved composite part", COMPOSITES, IPC BUSINESS PRESS LTD. HAYWARDS HEATH, GB, vol. 28, no. 9-10, 1 January 1997 (1997-01-01), pages 801-816, XP004096126, ISSN: 0010-4361, DOI: 10.1016/S1359-835X(97)00033-X * abstract * * page 802 - page 804 * ----- | 1-15 | |

TECHNICAL FIELDS
SEARCHED      (IPC)

The present search report has been drawn up for all claims

| Place of search | Date of completion of the search | Examiner |
|---|---|---|
| Munich | 17 April 2019 | Radev, Boyan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

page 3 of 3